# EUROPEAN PATENT SPECIFICATION

(11) **EP 2 042 091 B1**
(45) Date of publication and mention of the grant of the patent: **14.05.2014**
(21) Application number: 07117110.2
(22) Date of filing: 25.09.2007
(51) Int. Cl.: A61B 5/0215, A61B 5/03, A61M 25/09

(54) **Pressure sensing guidewire**
Drucksensor-Führungsdraht
Fil de guidage à détection de pression

(43) Date of publication of application: 01.04.2009
(73) Proprietor: RADI MEDICAL SYSTEMS AB, 754 50 Uppsala (SE)
(72) Inventor: Smith, Leif, 756 52, UPPSALA (SE)
(74) Representative: Brann AB

(56) References cited:
- EP-A- 1 774 905
- WO-A-2007/058826
- WO-A1-00/12004
- WO-A1-99/55225
- WO-A2-2005/086753
- US-A- 5 568 815
- US-A1- 2003 216 621
- US-A1- 2007 142 727
- US-B2- 6 585 660

## Description

### Field of the invention

The present invention relates to an assembly according to the preamble of the independent claim.

### Background of the invention

A sensor wire assembly is known from EP-1,774,905 assigned to the assignee of the present application for measuring a physiological variable, in particular pressure, inside a patient's body. The pressure sensor used in the EP-document is a miniaturized semiconductor sensor and one purpose of the assembly of EP-1,774,905 is to handle the electrical configurations of these miniaturized semiconductor sensors that are not always compatible with the transducer amplifiers in existing patient monitors. For instance, the miniaturized sensors often cannot operate over the entire range of excitation signal magnitudes and frequencies found among the various types of patient monitors. Thus, they cannot be connected directly to many of the patient monitors already in use. To be used with such existing monitors, a specialized interface must be placed between the sensor and the monitor. Such an arrangement necessitates additional circuitry on the interface and, because existing monitors have been designed to provide only limited amounts of power, the additional circuitry may require an independent source of electrical power. As a consequence, use of the newer miniaturized sensors often adds cost and complexity to the overall system.

In addition, because of the above limitations, these sensors must often be configured to generate an output signal which is proportional to the pressure sensed, but that is not related to the excitation signal, supplied to the sensor by the monitor, in a way that is directly usable by the physiology monitor, e.g. the sensitivity may be different. As discussed, this does not conform with the electrical format required by the many monitors that are commercially available and already in widespread use. As such, the newer sensors can only be used with specific signal conditioning and displaying units, thereby requiring additional equipment to be purchased. This is especially undesirable given the cost sensitivities so prevalent in today's health care environment.

US-5,568,815 discloses an interface circuit for interfacing a sensor to a patient monitor. The interface circuit includes a power supply circuit that receives an excitation power signal generated by the patient monitor, and derives therefrom unregulated and regulated supply voltages for use by the electrical components on the interface circuit. Further, the power supply circuit generates an appropriate sensor excitation signal. The interface circuit further includes receiving circuitry for receiving a sensor output signal generated by the sensor. A scaling circuit then scales that signal into a parameter signal that is proportional to the physiological condition detected by the sensor, and that is also proportional to the excitation power signal generated by the patient monitor.
An obvious drawback of the device of US-5,568,815 is that, in order to connect the sensor to the monitor, a separate additional unit in the form of the interface circuit is required.

A similar solution is disclosed in US-6,585,660 that relates to a signal conditioning device that interfaces a variety of sensor devices, such as guide wire-mounted pressure sensors, to physiology monitors. The signal conditioning device includes a processor for controlling a sensor excitation and signal conditioning circuitry within the signal conditioning device. The processor also supplies signals to an output stage on the signal conditioning device representative of processed sensor signals received by a sensor interface of the signal conditioning device. Power for the signal conditioning device processor is supplied by an excitation signal received from a physiology monitor that drives the output stage. In addition, a temperature compensating current source provides an adjustment current to at least one of a pair of resistive sensor elements to compensate for differences between temperature change upon the pair of resistive sensor elements, thereby facilitating nullifying temperature effects upon the resistive sensor elements.

The Association for the Advancement of Medical Instrumentation ("AAMI") has defined power requirements for physiology monitors and in particular the input/output connector to a sensor wire assembly must comply with the standard set by American National Standards Institute ("ANSI")/AAMI BP22-1994 (referred to as "BP22" in the following). According to the BP22-standard, an input/output connector arranged at the proximal end of a five line connector cable includes a pair of differential output signal lines. The output signal lines are driven by a sensor adapting circuitry's output digital to analog converters (discussed further herein below). The differential output signal, by way of example, operates at 5 µV /mmHg / V_{EXC}. An operation range of -150 µV/V to 1650 µV/V therefore represents a sensed pressure range of -30 to 330 mmHg. An exemplary resolution (minimum step) for the differential output signal is 0,2 mmHg.

A pressure measurement procedure when a pressure wire provided with a pressure sensor at its distal end is inserted into a patient's blood vessel, e.g. into the cardiac veins of the heart to measure local constrictions may last for several hours. The inventor has noted that variations and changes of the environmental air pressure in the operation room may have considerable impact on calculations based upon the measurement results received from the pressure sensor at the pressure wire inside the body in relation to environmental air pressure, which in particular may be significant if measurements are to be performed for longer time periods, e.g. several hours.

In order to fully appreciate the present invention a short background of the entire measurement procedure will be presented.
One important application of a pressure sensor wire is to be able to identify constrictions of coronary vessels, e.g. in the great cardiac vein, by performing so-called Fractional Flow Reserve-measurements (FFR). In short FFR is determined by obtaining the ratio between the pressure distally and proximally of a constriction. In practice a catheter is initially inserted and positioned with its distal part e.g. in the aorta. The distal part of the catheter is provided with a pressure sensor of a conventional type having a sensor typically positioned external to the patient's body and placed in fluid communication with the body cavity where the distal part of the catheter is positioned via a fluid-filled catheter line. Pressure variations within the body cavity are then indirectly communicated to the diaphragm at the pressure sensor by way of fluid contained in a lumen running within the entire catheter length and having a distal opening close to the distal end of the catheter. As such, the accuracy of such systems has suffered due to variations in hydrostatic pressure and other inconsistencies associated with the fluid column.
When the catheter is correctly positioned, the pressure sensor wire is inserted into another lumen of the catheter and advanced until the pressure sensor at the distal end of the pressure sensor wire is in, or close to, the distal opening of the lumen. The calibration of the pressure sensor of the pressure sensor wire in relation to the pressure measured by the catheter pressure sensor is then performed. When the calibration has been performed the pressure sensor wire is further advanced until its pressure sensor element is positioned distally of the constriction, and the assembly is then ready to measure the pressures and determine FFR.

Since the pressure sensor element is calibrated to the ambient pressure at the start of the measurement procedure, changes of the ambient pressure during the procedure may have significant effect of the accuracy of the calculated FFR as the measured catheter pressure reflects these changes whereas the pressure from the calibrated pressure sensor element does not. Thus, the catheter pressure sensor measures pressure in relation to the ambient pressure, whereas the pressure sensor of the pressure sensor wire measures the absolute pressure. During the calibration process the pressure sensor wire pressure is set equal to the catheter pressure sensor pressure.

Compensation for ambient pressure is known in other fields. One example is disclosed in WO 99/55225, which shows an implantable medical device for use with an implantable pressure sensor, wherein barometric pressure sensing values are used as reference pressure data when measuring pressure within a patient's body.

Thus, the object of the present invention is to achieve an improved pressure sensor assembly that generates reliable pressure measurement values irrespectively of external pressure variations, and that is easy to use and has a low over all cost.

### Summary of the invention

The above-mentioned object is achieved by the present invention according to the independent claim.

Preferred embodiments are set forth in the dependent claims.

According to the present invention a pressure sensor wire assembly for measuring pressure inside a body of a patient comprises a pressure sensor wire with a pressure sensor element at its distal portion for measuring pressure and to generate a pressure sensor signal in response of said pressure. The pressure sensor wire is adapted to be inserted into the body in order to position the sensor element within the body. Furthermore, a sensor signal adapting circuitry is an integrated part of the assembly, wherein the pressure sensor signal is applied to the adapting circuitry that is adapted to automatically generate an output pressure signal, related to the sensor signal, in a standardized format such that the measured pressure is retrievable by an external physiology monitor. The assembly further comprises an external pressure sensor arranged in the assembly to measure the pressure outside the patient's body and to generate external pressure values in dependence thereto. The external pressure values are applied to a pressure compensation means, in said assembly, adapted to generate a compensation value reflecting the external pressure variation during a measurement procedure, wherein the output pressure signal is compensated by said compensation value prior to the output pressure signal being applied to the external physiology monitor. The assembly further comprises a transceiver unit and a communication unit, where the transceiver unit is adapted to wirelessly communicate via a communication signal with said communication unit. The communication unit is connected to the external physiology monitor, in order to transfer the output pressure signal to the external physiology monitor, and the external pressure sensor is arranged in said communication unit.

The transceiver unit is adapted to be arranged outside the patient's body, and the pressure sensor wire is adapted to be connected, at its proximal end, to the transceiver unit which is provided with an elongated aperture adapted to receive the proximal end of the pressure sensor wire. This aperture is at its inner surface provided with a number of electrical connecting surfaces to be connected to electrode surfaces at the proximal end of the pressure sensor wire when inserted into the aperture. The transceiver unit is further provided with wire fastening means to firmly fixate the wire when correctly inserted into the aperture. In addition, the transceiver unit is adapted to be used as a torque device when guiding the pressure sensor wire during insertion into a patient and when the wire is fixated to the transceiver unit.

Hence, according to the invention a pressure monitoring sensor, i.e. a barometer, measures external pressure when the procedure of determining the internal pressure is initiated and determines a compensation value based upon the initial external pressure value and the present value, in order to compensate for pressure variation occurring during the whole procedure. A pressure difference value is determined by determining the difference between the present pressure value, i.e. the pressure value present when the pressure measurement is performed, and the initial pressure value and the measured internal pressure value is then compensated by the pressure difference value.

### Short description of the appended drawings

Figure 1 schematically illustrates a pressure sensor assembly according to prior art.
Figure 2 schematically shows a block diagram of a wire connector of a pressure sensor assembly according to prior art.
Figure 3 schematically illustrates the present invention.
Figure 4 schematically shows the transceiver unit of the present invention.
Figure 5 schematically shows a block diagram of the transceiver unit of the present invention.
Figure 6 schematically shows a block diagram of the communication unit of the present invention.

### Detailed description of preferred embodiments of the invention

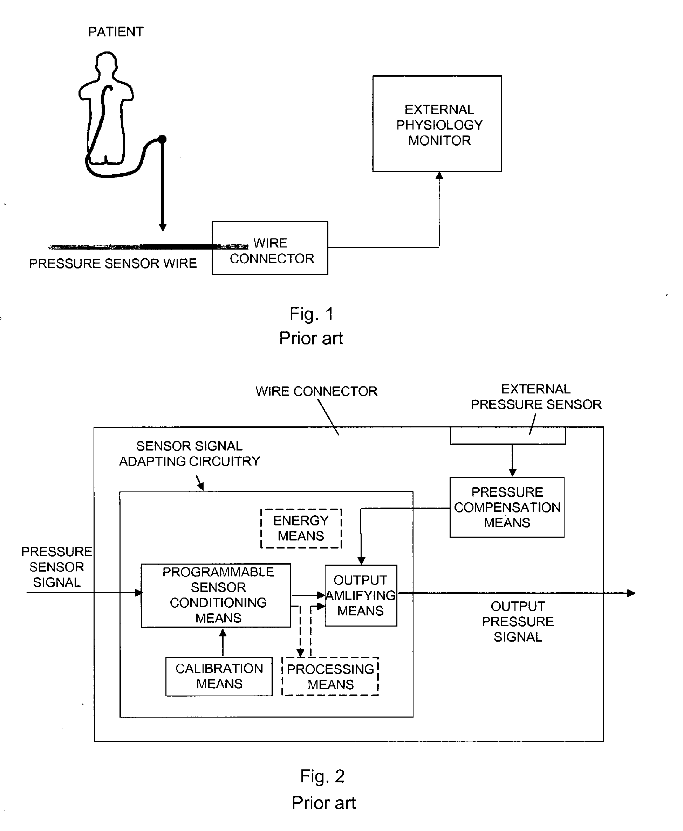

To facilitate description of the details of the present invention, a prior art assembly will first be described. Figure 1 schematically illustrates a pressure sensor wire assembly for measuring pressure in a body of a patient according to prior art, and figure 2 shows a block diagram schematically illustrating a wire connector as known in the art.

Figure 2 shows a pressure sensor wire assembly for measuring pressure inside a body of a patient. As is known in the art, such an assembly can comprise a pressure sensor element, not shown in the figure, for measuring pressure and to generate a pressure sensor signal in response of the measured pressure, a pressure sensor wire (fig. 1) having the pressure sensor element at its distal portion, and adapted to be inserted into the body of a patient in order to position the sensor element within the body. The assembly further comprises a sensor signal adapting circuitry, being an integrated part of the assembly, wherein the pressure sensor signal is applied to the adapting circuitry that is adapted to automatically generate an output pressure signal, related to the sensor signal, in a standardized format such that the measured pressure is retrievable by an external physiology monitor (fig. 1). The sensor signal adapting circuitry comprises a programmable sensor conditioning unit, a calibration unit, being a storage means into which calibration data may be supplied, stored and altered, e.g. an electrically erasable programmable read-only memory (EEPROM), energy means and an output amplifying unit.

The programmable sensor conditioning unit is preferably a PGA309 programmable analog sensor conditioner (available from Texas Instruments Inc.) specifically designed for bridge sensors. The PGA309 is particularly designed for resistive bridge sensor applications and contains three main gain blocks for scaling differential input bridge sensor signals. Hence, as discussed in the above, a signal representing the measured physiological variable may be adapted such that a signal in a format expected by the monitor is provided. This signal format is determined by the reference voltage supplied to the sensor signal adapting circuitry and the actual value of the signal measured by the sensor. The PGA309 can be configured for use with an internal or external voltage reference. An internal reference voltage of e.g. +2,5V is supplied to the PGA309 from the energy means.

Thus, the conditioning means generates an analog output voltage signal related to the sensor signal such that the measured physiological variable, i.e. the pressure, may be retrieved by the external device.

Since each sensor element is an individual item with its own characteristics, each sensor assembly comprises a calibration means, preferably an electrically erasable programmable read-only memory (EEPROM) which contains individual calibration data obtained during calibration of the sensor element performed for each individual sensor wire assembly. The calibration is performed in connection with manufacture of the pressure sensor wire. Calibration data takes into account parameters such as voltage offsets and temperature drift, etc.

The bridge pressure sensor is preferably energized from the PGA309 via an excitation voltage V_{EXC}, generated by the PGA309 circuit. As an alternative the pressure sensor may be energized from a separate energy source, e.g. a battery or a capacitor means, or from an external power supply, e.g. an external mains supply via the monitor.

For a given excitation voltage V_{EXC}, e.g. generated by the PGA309 circuit, the output voltage (V_{IN1} - V_{IN2}) of the bridge is a voltage proportional to the pressure applied to the sensor. Hence, the sensor output voltage (V_{IN1} - V_{IN2}) (sensor signal in figure 2) of the bridge is proportional to the pressure applied to the sensor, which for a given pressure will vary with the applied excitation voltage. This sensor output voltage is preferably compensated for temperature variation at the site of the sensor and the compensated sensor output voltage is applied to the PGA309 circuit. The PGA309 circuit also includes gain blocks for adjusting the output signal from that circuit and are used in addition to the output amplifying means mentioned above.

Preferably a microprocessor (e.g. a PIC16C770 or a nRF24E1, shown with dashed lines in figure 2) may further be employed to process and adapt the analog output voltage V_{OUT} of the conditioned sensor, which output voltage is supplied via the PGA309 programmable analog sensor conditioner. The analog output signal from the PGA309 circuit is A/D-converted prior to being applied to the processing means. To adapt the sensor signal to the BP22 signal standard, it may be necessary to process the sensor signal further before it is applied to the physiology monitor. For instance a multiplying digital-analog converter (DAC) which possibly is comprised in the processing means is supplied with digital data (e.g. a 12-bit word) representing the signal measured by the sensor element and the reference voltage.

In the prior art device of figure 2, a proximal portion of the pressure sensor wire is adapted to be connected. The wire connector is in turn connected to the external physiology monitor via a connecting cable, not shown in figure 2. The wire connector includes the above-mentioned sensor signal adapting circuitry.

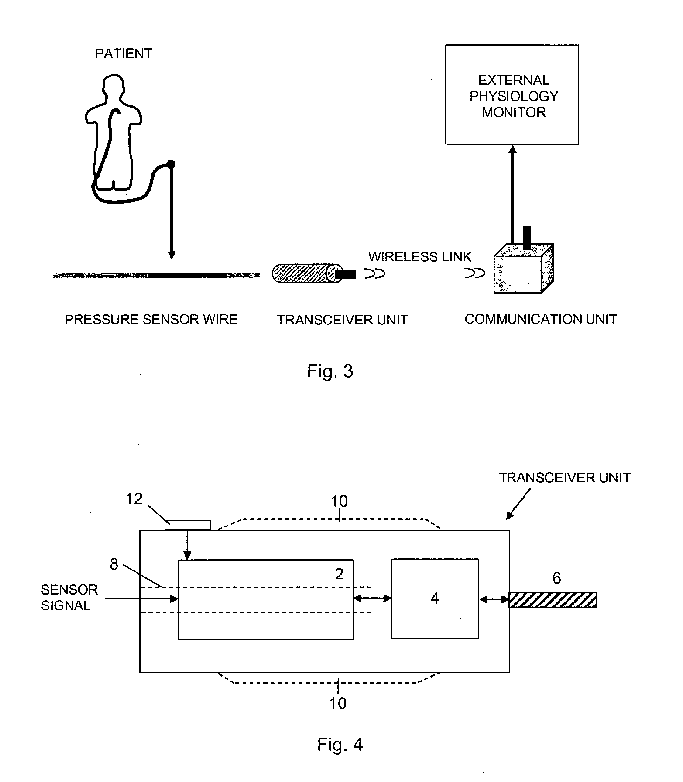

Figure 3 schematically illustrates the present invention, where the assembly comprises a transceiver unit and a communication unit. Figure 4 shows the transceiver unit of the present invention. The pressure sensor wire is adapted to be connected, at its proximal end, to the transceiver unit that is adapted to wirelessly communicate via a communication signal with the communication unit, and the communication unit is in turn connected to the external physiology monitor, in order to transfer the output pressure signal to the external physiology monitor. The assembly includes the above-mentioned sensor signal adapting circuitry and calibration means.

The assembly further comprises an external pressure sensor arranged at the assembly to measure the pressure outside the patient's body and to generate external pressure values in dependence thereto. The external pressure values are applied to a pressure compensation means, in the assembly, adapted to generate a compensation value reflecting the external pressure variation during a measurement procedure, and the output pressure signal is compensated by the compensation value prior to the output pressure signal being applied to the external physiology monitor.

A person skilled in the art of the pressure sensors will be able to identify numerous pressure sensors applicable as external pressure sensors to be used in the pressure wire assembly according to the present invention. In the following two exemplary sensors will be described.

The external pressure sensor may be a SCP1000-D01/D11 digital absolute pressure sensor by VTI Technologies, intended for barometric pressure measurement and altimeter applications for 30kPa...120kPa and -20°C...70°C measuring ranges. The pressure and temperature output data is calibrated and compensated internally and the communication between the SCP1000 and its host micro-controller is realized using SPI or I²C interfaces. SCP1000 is comprised of a VTI's 3D MEMS capacitive sensing element, a dedicated low power CMOS interface ASIC with on-chip calibration memory, 4 preset measuring modes and a LCP (Liquid Crystal Plastic) MID (Molded Interconnect Device) housing. The component is a surface mountable device incorporating a circular vertical wall for easy waterproof sealing.

Another applicable sensor is a pressure sensor by Honeywell Sensing and Control, Inc. in the 24PC Series that contain sensing elements that consist of four piezoresistors buried in the face of a thin, chemically-etched silicon diaphragm. A pressure change causes the diaphragm to flex, inducing a stress in the diaphragm and the buried resistors. The resistor values change in proportion to the stress applied and produce an electrical output. These sensors are small, low cost and reliable. They feature excellent repeatability, high accuracy and reliability under varying environmental conditions. In addition, they feature highly consistent operating characteristics from one sensor to the next, and interchangeability without recalibration.

When a measurement procedure is initiated an initial external pressure value is determined as being the external pressure value measured initially, and the compensation means is adapted to generate the compensation value in relation to the difference between the present value and the initial external pressure value. Thus, each time a measurement is performed, i.e. for each pressure value obtained by the pressure sensor wire and generated by the assembly during a measurement procedure, the pressure value is compensated for any variation of the external pressure by adding or subtracting the pressure value by the compensation value.

The pressure compensation means comprises a control means and a storage means, not shown in the figures, and the generated external pressure values are stored in said storage means prior to being used by the control means to determine the compensation value.

The wireless communication, where the communication signal is a radio frequency signal, will now be described in detail. The wireless communication is performed by using an established communication protocol, e.g. Bluetooth. Although the transceiver unit and the communication unit are described in connection with the use of a radio frequency signal it should be appreciated that relevant features would be equally applicable in case any alternative communication signals is used, e.g. optical or magnetic signals.

With references to figures 3 and 4, the communication module is connected to an antenna 6. In the figures the antenna is illustrated as protruding outside the transceiver unit but may, as in an alternative, be integrated into the housing of the transceiver unit. The pressure sensor wire is adapted to be inserted into an elongated aperture 8 of the transceiver unit. The aperture is at its inner surface provided with a number of electrical connecting surfaces (not shown) to be connected to electrode surfaces at the proximal end of the pressure sensor wire when inserted into the aperture 8. The transceiver unit is further provided with wire fastening means (not shown) to firmly fixate the wire when correctly inserted into the aperture.

According to a preferred embodiment the transceiver unit is adapted to receive the proximal end to the pressure sensor wire having an outer diameter of 0,35 mm, i.e. the inner diameter of the elongated aperture 8 is slightly larger than 0,35 mm.

When the pressure sensor wire is fixated to the transceiver unit the unit may be used as a torque device when guiding the pressure sensor wire during insertion into a patient. Preferably the transceiver unit is provided with guiding means 10, e.g. in the form of one or many elongated ribs on the outer surface of the transceiver unit, or by providing the transceiver unit with a roughened surface.

The pressure sensor wire may be fixated to the transceiver unit such that as the transceiver unit is rotated along its longitudinal axis the sensor wire is also rotated, which often is necessary in order to guide the sensor wire during the insertion procedure. As an alternative, the sensor wire is fixated to the transceiver unit in such way that the sensor wire may be rotated in relation to the transceiver unit. The rotation of the sensor wire is then achieved by firmly holding the transceiver unit by one hand and by rotating the sensor wire by the other hand.

The transceiver unit is preferably activated and initiated via an activation button 12 arranged at the housing of the unit. The activation button is preferably mechanically activated.

The transceiver unit comprises energy means to energize the transceiver unit and the circuitry of the connected pressure sensor wire. The energy means is preferably a battery or a capacitor that e.g. may be included in the sensor signal adapting circuitry.

The pressure sensor wire as well as the transceiver unit are preferably disposable units that should be possible to sterilise prior to use.

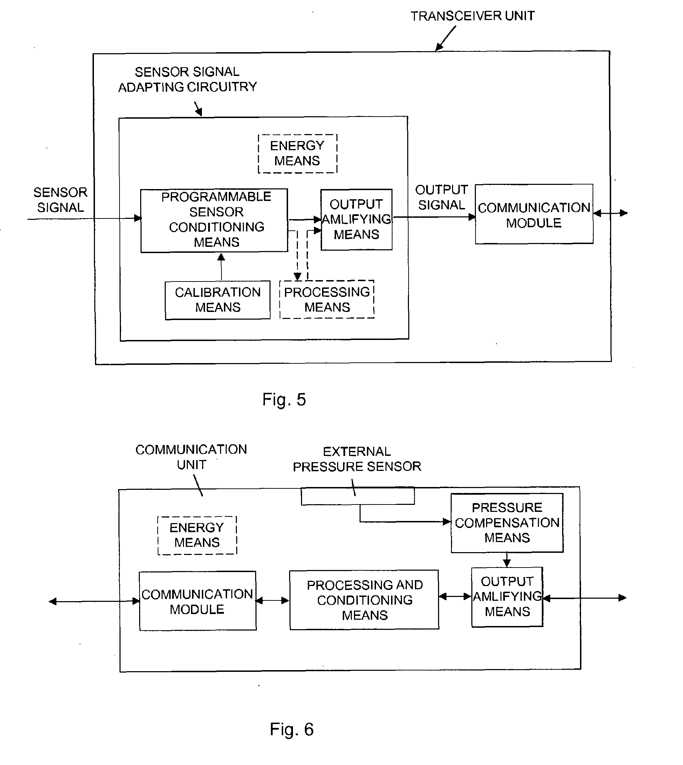

The transceiver unit comprises a first communication module to handle the radio frequency communication with the communication unit that is provided with a second communication module (see figures 6 and 8).

When the pressure sensor wire has been inserted into the transceiver unit and the communication unit is connected to the external device the system is ready for use.
By pressing the activation button on the transceiver unit it is activated and will then try to establish a radio link connection with the communication unit. This is preferably performed by a conventional handshake procedure in order to identify the transceiver unit. The system is now ready to receive measured sensor data.

In the present invention, the external pressure sensor and the pressure compensation means are arranged in the communication unit. Figures 5 and 6 schematically show block diagrams of the transceiver unit and of the communication unit, respectively, of the present invention.

With regard to the features and function of the sensor signal adapting circuitry of the present invention it is referred to the description in connection with figure 2.

In one embodiment, the adaptation of the sensor signal to the standard, e.g. BP22 signal standard, is performed in the transceiver unit, and in particular in the sensor signal adapting circuitry (see figure 5). However, this adaptation, in its entirety or only in parts, may, as an alternative, instead be performed by a corresponding circuitry arranged in the communication unit. This embodiment is schematically illustrated in figure 6. The wirelessly transmitted pressure sensor values would then be in the form of "raw" measured data that would be conditioned by a processing and conditioning means in the communication unit in order to be in a correct format to be supplied to the external system according to a prescribed standard format.

Thus, on embodiment is illustrated by figures 5 and 6, where the processing and conditioning means should be omitted in figure 6. Another embodiment is illustrated by figures 5 and 6 where instead the programmable sensor conditioning means should be omitted in figure 5.

The external pressure sensor is either energized by the energy means within the communication unit, or energized via the external monitor and/or via external mains supply. The energy means may be e.g. a battery or capacitor.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Pressure sensor wire assembly for measuring pressure inside a body of a patient, the assembly comprises:
a pressure sensor element for measuring pressure and to generate a pressure sensor signal in response of said pressure;
a pressure sensor wire having said pressure sensor element at its distal portion, and adapted to be inserted into the body in order to position the sensor element within the body,
a sensor signal adapting circuitry, being an integrated part of said assembly, wherein the pressure sensor signal is applied to the adapting circuitry that is adapted to automatically generate an output pressure signal, related to the sensor signal, in a standardized format such that the measured pressure is retrievable by an external physiology monitor,
the assembly further comprises an external pressure sensor arranged in the assembly to measure the pressure outside the patient's body and to generate external pressure values in dependence thereto, the external pressure values are applied to a pressure compensation means, in said assembly, adapted to generate a compensation value reflecting the external pressure variation during a measurement procedure, wherein the output pressure signal is compensated by said compensation value prior to the output pressure signal being applied to the external physiology monitor, and that the assembly further comprises a transceiver unit and a communication unit, and that said transceiver unit is adapted to wirelessly communicate via a communication signal with said communication unit, the communication unit is connected to the external physiology monitor, in order to transfer the output pressure signal to the external physiology monitor, and that the external pressure sensor is arranged in said communication unit,
**characterized in that**
the transceiver unit is adapted to be arranged outside the patient's body, and the pressure sensor wire is adapted to be connected, at its proximal end, to said transceiver unit which is provided with an elongated aperture (8) adapted to receive the proximal end of the pressure sensor wire, said aperture is at its inner surface provided with a number of electrical connecting surfaces to be connected to electrode surfaces at the proximal end of the pressure sensor wire when inserted into the aperture, and that the transceiver unit is further provided with wire fastening means to firmly fixate the wire when correctly inserted into the aperture, and that the transceiver unit is further adapted to be used as a torque device when guiding the pressure sensor wire during insertion into a patient and when the wire is fixated to the transceiver unit.

2. Pressure sensor wire assembly according to claim 1, wherein an initial external pressure value is determined as the external pressure value measured when a measurement procedure is initiated, and the compensation means is adapted to generate said compensation value in relation to the difference between the present and initial external pressure values.

3. Pressure sensor wire assembly according to claim 1 or 2, wherein the wireless communication is performed by using an established communication protocol, e.g. Bluetooth.

4. Pressure sensor wire assembly according to any of claims 1-3, wherein said pressure compensation means comprises a control means and a storage means, and that the generated external pressure values are stored in said storage means.

5. Pressure sensor wire assembly according to any of claims 1-4, wherein said pressure compensation means is arranged in said communication unit.

6. Pressure sensor wire assembly according to any of claims 1-5, wherein the proximal end of the pressure sensor wire having an outer diameter of 0.35 mm, and the inner diameter of the elongated aperture (8) is slightly larger than 0.35 mm.

## Patentansprüche

1. Drucksensordrahtanordnung zum Messen des Drucks im Inneren eines Körpers eines Patienten, wobei die Anordnung aufweist:
ein Drucksensorelement zum Messen des Drucks und zum Erzeugen eines Drucksensorsignals ansprechend auf den Druck;
einen Drucksensordraht mit dem Drucksensorelement an seinem distalen Abschnitt, der geeignet ist, in den Körper eingesetzt zu werden, um das Sensorelement innerhalb des Körpers zu positionieren,
eine Sensorsignal-Anpassungsschaltung, die ein integrierter Teil der Anordnung ist, wobei das Drucksensorsignal der Anpassungsschaltung zugeführt wird, die geeignet ist, um automatisch ein Ausgangsdrucksignal, das sich auf das Sensorsignal bezieht, in einem standardisierten Format zu erzeugen, so dass der gemessene Druck durch eine externe Physiologie-Überwachungseinrichtung abrufbar ist,
wobei die Anordnung ferner einen externen Drucksensor aufweist, der in der Anordnung angeordnet ist, um den Druck außerhalb des Körpers des Patienten zu messen und in Abhängigkeit davon externe Druckwerte zu erzeugen, wobei die externen Druckwerte einer Druckkompensationseinrichtung in der Anordnung zugeführt werden, die geeignet ist, einen Kompensationswert zu erzeugen, der die externe Druckänderung während eines Messverfahrens widerspiegelt, wobei das Ausgangsdrucksignal durch den Kompensationswert kompensiert wird, bevor das Ausgangsdrucksignal der externen Physiologie-Überwachungseinrichtung zugeführt wird, und wobei die Anordnung ferner eine Transceivereinheit und eine Kommunikationseinheit aufweist und die Transceivereinheit geeignet ist, über ein Kommunikationssignal drahtlos mit der Kommunikationseinheit zu kommunizieren, wobei die Kommunikationseinheit mit der externen Physiologie-Überwachungseinrichtung verbunden ist, um das Ausgangsdrucksignal an die externe Physiologie-Überwachungseinrichtung zu übertragen, und wobei der externe Drucksensor in der Kommunikationseinheit angeordnet ist,
**dadurch gekennzeichnet, dass**
die Transceivereinheit geeignet ist, außerhalb des Körpers des Patienten angeordnet zu werden, und der Drucksensordraht geeignet ist, an seinem proximalen Ende mit der Transceivereinheit verbunden zu werden, die mit einer länglichen Öffnung (8) versehen ist, die geeignet ist, das proximale Ende des Drucksensordrahts aufzunehmen, wobei die Öffnung an ihrer inneren Oberfläche mit einer Anzahl elektrischer Anschlussoberflächen versehen ist, die mit Elektrodenoberflächen an dem proximalen Ende des Drucksensordrahts verbunden werden sollen, wenn er in die Öffnung eingesetzt ist, und dass die Transceivereinheit ferner mit Drahtbefestigungseinrichtungen versehen ist, um den Draht fest zu fixieren, wenn er korrekt in die Öffnung eingesetzt ist, und dass die Transceivereinheit ferner geeignet ist, als eine Drehmomentvorrichtung verwendet zu werden, wenn der Drucksensordraht während des Einsetzens in einen Patienten geführt wird und wenn der Draht an einer Transceivereinheit fixiert ist.

2. Drucksensordrahtanordnung nach Anspruch 1, wobei ein anfänglicher externer Druck als der externe Druckwert bestimmt wird, der gemessen wird, wenn ein Messverfahren eingeleitet wird, und die Kompensationseinrichtung geeignet ist, den Kompensationswert in Bezug auf die Differenz zwischen den aktuellen und anfänglichen externen Druckwerten zu erzeugen.

3. Drucksensordrahtanordnung nach Anspruch 1 oder 2, wobei die drahtlose Kommunikation unter Verwendung eines etablierten Kommunikationsprotokolls, z.B. Bluetooth, durchgeführt wird.

4. Drucksensordrahtanordnung nach einem der Ansprüche 1 - 3, wobei die Druckkompensationseinrichtung eine Steuereinrichtung und eine Speichereinrichtung aufweist und die erzeugten externen Druckwerte in der Speichereinrichtung gespeichert werden.

5. Drucksensordrahtanordnung nach einem der Ansprüche 1 - 4, wobei die Druckkompensationseinrichtung in der Kommunikationseinheit angeordnet ist.

6. Drucksensordrahtanordnung nach einem der Ansprüche 1 - 5, wobei das proximale Ende des Drucksensordrahts einen Außendurchmesser von 0,35 mm hat und der Innendurchmesser der länglichen Öffnung (8) ein wenig größer als 0,35 mm ist.

## Revendications

1. Ensemble fil de capteur de pression pour mesurer la pression à l'intérieur d'un corps d'un patient, l'ensemble comprend :
un élément de capteur de pression pour mesurer la pression et générer un signal de capteur de pression en réponse à ladite pression ;
un fil de capteur de pression ayant ledit élément de capteur de pression au niveau de sa partie distale, et adapté pour être inséré dans le corps afin de positionner l'élément de capteur à l'intérieur du corps,
un ensemble de circuits d'adaptation de signal de capteur, faisant partie intégrante dudit ensemble, dans lequel le signal de capteur de pression est appliqué à l'ensemble de circuits d'adaptation qui est adapté pour générer automatiquement un signal de pression de sortie, lié au signal de capteur, dans un format normalisé de sorte que la pression mesurée soit récupérable par un moniteur de physiologie externe,
l'ensemble comprend en outre un capteur de pression externe agencé dans l'ensemble pour mesurer la pression à l'extérieur du corps du patient et pour générer des valeurs de pression externe qui en dépendent, les valeurs de pression externe sont appliquées à un moyen de compensation de pression, dans ledit ensemble, adapté pour générer une valeur de compensation reflétant la variation de pression externe au cours d'une procédure de mesure, dans lequel le signal de pression de sortie est compensé par ladite valeur de compensation avant que le signal de pression de sortie ne soit appliqué au moniteur de physiologie externe, et l'ensemble comprend en outre une unité émetteur-récepteur et une unité de communication, et ladite unité émetteur-récepteur est adaptée pour communiquer de manière sans fil via un signal de communication avec ladite unité de communication, l'unité de communication est connectée au moniteur de physiologie externe, afin de transférer le signal de pression de sortie au moniteur de physiologie externe, et le capteur de pression externe est agencé dans ladite unité de communication,
**caractérisé en ce que**
l'unité émetteur-récepteur est adaptée pour être agencée à l'extérieur du corps du patient, et le fil de capteur de pression est adapté pour être connecté, à son extrémité proximale, à ladite unité émetteur-récepteur qui est pourvue d'une ouverture allongée (8) adaptée pour recevoir l'extrémité proximale du fil de capteur de pression, ladite ouverture est au niveau de sa surface intérieure pourvue d'un nombre de surfaces de connexion électriques à connecter à des surfaces d'électrode au niveau de l'extrémité proximale du fil de capteur de pression lorsqu'il est inséré dans l'ouverture, et **en ce que** l'unité émetteur-récepteur est en outre pourvue d'un moyen d'attache de fil pour fixer solidement le fil lorsqu'il est inséré correctement dans l'ouverture, et **en ce que** l'unité émetteur-récepteur est en outre adaptée pour être utilisée comme un dispositif de couple lors du guidage du fil de capteur de pression au cours de l'insertion dans un patient et lorsque le fil est fixé à l'unité émetteur-récepteur.

2. Ensemble fil de capteur de pression selon la revendication 1, dans lequel une valeur de pression externe initiale est déterminée comme la valeur de pression externe mesurée lorsqu'une procédure de mesure est initiée, et le moyen de compensation est adapté pour générer ladite valeur de compensation relativement à la différence entre les valeurs de pression externe actuelle et initiale.

3. Ensemble fil de capteur de pression selon la revendication 1 ou 2, dans lequel la communication sans fil est effectuée en utilisant un protocole de communication établi, par exemple, Bluetooth.

4. Ensemble fil de capteur de pression selon l'une quelconque des revendications 1 à 3, dans lequel ledit moyen de compensation de pression comprend un moyen de commande et un moyen de stockage, et les valeurs de pression externe générées sont stockées dans ledit moyen de stockage.

5. Ensemble fil de capteur de pression selon l'une quelconque des revendications 1 à 4, dans lequel ledit moyen de compensation de pression est agencé dans ladite unité de communication.

6. Ensemble fil de capteur de pression selon l'une quelconque des revendications 1 à 5, dans lequel l'extrémité proximale du fil de capteur de pression ayant un diamètre extérieur de 0, 35 mm, et le diamètre intérieur de l'ouverture allongée (8) est légèrement supérieur à 0,35 mm.
